# EUROPEAN PATENT APPLICATION

(11) **EP 4 576 113 A1**
(43) Date of publication of application: **25.06.2025**
(21) Application number: 24219282.1
(22) Date of filing: 12.12.2024
(51) Int. Cl.: G16H 40/20, G16H 50/70

(54) **PERSONNEL ON-DUTY STATUS EARLY-WARNING METHOD, APPARATUS, DEVICE, SYSTEM AND MEDIUM**

(30) Priority: 19.12.2023 CN 202311755985
(71) Applicant: Kingfar International Inc., Beijing 100085 (CN)
(72) Inventor: ZHAO, Qichao, Beijing (CN); YANG, Ran, Beijing (CN)
(74) Representative: Meyer-Dulheuer MD Legal Patentanwälte PartG mbB

(57) **Abstract**

Provided is a personnel on-duty status early-warning method, apparatus, device, system and medium. The method includes: continuously collecting human body data of personnel in an actual working environment; determining on-duty status of the personnel corresponding to the collected human body data based on the obtained human body data by using a state detection model, the state detection model is obtained by pre-training a preset model based on a training dataset, and the training dataset includes human body data and corresponding on-duty status annotation information; performing on-duty status monitoring on the personnel based on the on-duty status determined for the personnel; and outputting early-warning prompt information in response to the on-duty status of the personnel during the on-duty status monitoring meeting a preset early-warning condition. The method can improve the detection accuracy of the personnel on-duty status.

## Description

### TECHNICAL FIELD

The present disclosure relates to the fields of human factors engineering, artificial intelligence and safety, and in particular to a personnel on-duty status early-warning method, apparatus, device, system and medium.

### BACKGROUND

Self-control resource theory holds that self-control is a limited resource. Individuals may consume their own resources when performing self-control, resulting in poor performance in subsequent self-control tasks. Transient state refers to a short-term, temporary state or event, such as emotional fluctuations or stress responses, etc. Based on the self-control resource theory, transient state may occupy the individuals' resources such as emotions and cognition, leading to the depletion of limited self-control resources and damage to health within a certain period of time.

For practitioners, especially for some high-risk personnel such as those employed in the numerical control, chemical engineering, construction and other industries, cognitive resource depletion may occur under transient state, resulting in misoperations. Monitoring on-duty physical and mental status of the high-risk personnel and providing timely warnings can effectively reduce the rate of misoperations caused by human factors, and may even avoid major risky operations, thereby ensuring the personal safety of personnel.

### SUMMARY

The present disclosure provides a personnel on-duty status early-warning method, apparatus, device, system and medium.

According to a first aspect, embodiments of the present disclosure provide a personnel on-duty status early-warning method, adopting the following technical solutions.

A personnel on-duty status early-warning method, including: continuously collecting human body data of personnel in an actual working environment; collecting human body data of personnel in an actual working environment continuously; determining on-duty status of the personnel corresponding to the human body data based on the obtained human body data by using a status detection model, the state detection model is obtained by pre-training a preset model based on a training dataset, and the training dataset includes human body data and its corresponding on-duty status annotation information; performing on-duty status monitoring on the personnel based on the on-duty status determined for the personnel; and outputting early-warning prompt information if the on-duty status of the personnel during the on-duty status monitoring meets a preset early-warning condition.

By adopting the above technical solutions, based on the obtained human body data of the personnel in the actual working environment, the on-duty status of the personnel may be determined by using the detection model. The state detection model is obtained by pre-training the preset model based on the training dataset, which can improve the detection accuracy of the on-duty status of the personnel. The training dataset includes human body data and corresponding on-duty status annotation information, and the accurate subjective-perception information of the personnel can be obtained through the on-duty status annotation information, which improves the accuracy of performing the on-duty status monitoring on the personnel based on the on-duty status determined for the personnel. If the on-duty status of the personnel during the on-duty status monitoring meets the preset early-warning condition, the early-warning prompt information is output, thereby avoiding the operation errors of the personnel and improving the life safety of the personnel.

In one or more embodiments, human body data collected in a simulated working environment, and corresponding on-duty status annotation information and on-duty information, and/or, the training dataset comprises historical human body data collected in an actual working environment and corresponding on-duty status annotation information and on-duty information. The on-duty information comprises years of work and types of work.

In one or more embodiments, the method further includes: determining work tasks, interactive elements, control methods and feedback mechanisms; building the simulated working environment based on the work tasks, interactive elements, control methods and feedback mechanisms; generating task instructions and send the task instructions to a terminal device of tested personnel to prompt the tested personnel to execute the work tasks corresponding to the task instructions in the simulated working environment; obtaining human body data corresponding to the tested personnel when executing the work tasks and its corresponding on-duty status annotation information and on-duty information; generating the training dataset based on the human body data corresponding to the tested personnel and its corresponding on-duty status annotation information and on-duty information; and training the preset model through the training dataset to obtain the state detection model.

By adopting the above technical solutions, a simulated working environment is built, and the tested personnel executes the work tasks in the simulated working environment, so that the human body data of the tested personnel in the simulated working environment and corresponding on-duty status annotation information and on-duty information may be obtained, which improves the accuracy of determining the training dataset. The preset model is trained through the training dataset, the state detection model with more accurate detection is obtained, thereby improving the test accuracy of the state detection model.

In one or more embodiments, the training dataset includes a training set, a test set and a validation set. The training the preset model through the training dataset to obtain the state detection model, includes: performing initial training on the preset model to obtain an initial model based on the training set, wherein the preset model is obtained by pre-training based on an initial training dataset, and the initial training dataset at least comprises human body data of ordinary personnel and corresponding on-duty status annotation information; performing metric testing on the initial model based on the test set to obtain an initial status detection model; and verifying the initial status detection model based on the validation set to obtain the status detection model.

By adopting the above technical solutions, the preset model is initially trained, tested and verified in sequence through the training set, test set and validation set. The obtained state detection model is more in line with the physical state of the personnel in the working environment, thereby improving the detection accuracy of the state detection model.

In one or more embodiments, the obtaining human body data corresponding to the tested personnel when executing the work tasks and its corresponding on-duty status annotation information and on-duty information, includes: during the process of the tested personnel executing the work tasks, obtaining the human body data corresponding to the tested personnel, a working duration of the tested personnel, and limb movement data of the tested personnel; inputting the human body data corresponding to the tested personnel into the preset model to generate simulated on-duty status of the tested personnel; determining whether the simulated on-duty status is an abnormal state, whether the working duration reaches a preset duration, and/or whether limb movements of the tested personnel are qualified based on the limb movement data; and generating the on-duty status annotation information and on-duty information of the tested personnel based on the determination result.

By adopting the above technical solutions, determining whether the simulated on-duty status is an abnormal state, whether the working duration reaches the preset duration, and/or whether the limb movements of the tested personnel are qualified based on the limb movement data, and generating the on-duty status annotation information and on-duty information of the tested personnel based on the determined result can improve the accuracy of the on-duty status annotation information.

In one or more embodiments, the on-duty status annotation information at least includes fatigue data and/or emotion data. The generating the on-duty status annotation information of the tested personnel, includes: sending a fatigue assessment scale and/or an emotion scale to the terminal device of the tested personnel through a subjective questionnaire; receiving fatigue data of the fatigue assessment scale and/or emotion data of the emotion scale corresponding to the tested personnel.

By adopting the above technical solutions, through the fatigue assessment scale and the emotion scale, the accurate fatigue data and emotion data of the tested personnel may be obtained, which improves the accuracy of the on-duty status annotation information, and then the subjective on-duty status of the tested personnel can be evaluated in multiple aspects through the fatigue data and emotion data.

In one or more embodiments, the determining on-duty status of the personnel corresponding to the human body data based on the obtained human body data by using a status detection model, includes: performing data pre-processing and feature extraction on the human body data; inputting the extracted features into the status detection model to determine fatigue data and emotion data corresponding to the human body data, wherein the on-duty status annotation information in the training dataset corresponding to the status detection model comprises fatigue data and emotion data; and inputting the fatigue data and emotion data into a preset classification model to obtain the on-duty status of the personnel corresponding to the human body data, wherein the on-duty status comprises a cognitive load level, a fatigue level and an emotion level.

By adopting the above technical solutions, performing data pre-processing, feature extraction and state detection on the human body data may determine the fatigue data and emotion data corresponding to the human body data. Inputting the fatigue data and emotion data into the preset classification model improves the accuracy of determining the on-duty status of the personnel.

In one or more embodiments, the determining on-duty status of the personnel corresponding to the human body data based on the obtained human body data by using a status detection model, includes: performing data pre-processing and feature extraction on the human body data; and inputting the extracted features into the status detection model to determine the on-duty status of the personnel corresponding to the human body data, wherein the on-duty status annotation information in the training dataset corresponding to the status detection model comprises cognitive load level annotation information, fatigue level annotation information and emotion level annotation information.

By adopting the above technical solutions, through performing data pre-processing and feature extraction on the physiological data to be detected and inputting the extracted features into the state detection model, the cognitive load level, fatigue level and emotion level of the personnel can be accurately determined.

In one or more embodiments, the performing on-duty status monitoring on the personnel based on the on-duty status determined for the personnel, includes: determining whether the cognitive load level, fatigue level and emotion level exceed respective level thresholds; and determining that the on-duty status of the personnel does not meet the preset early-warning condition in response to all of the cognitive load level, fatigue level and emotion level do not exceed respective level thresholds, or determining that the on-duty status of the personnel meets the preset early-warning condition in response to any one of the cognitive load level, fatigue level and emotion level exceeds respective level threshold.

By adopting the above technical solutions, according to the comparison results of the cognitive load level, fatigue level and emotion level with the respective corresponding level thresholds, it may be determined whether the on-duty status of the high-risk personnel meets the preset early-warning condition, thereby improving the determination efficiency and accuracy.

In one or more embodiments, the human body data includes a variety of physiological data and/or electroencephalogram data.

According to a second aspect, embodiments of the present disclosure provide a personnel on-duty status early-warning apparatus, adopting the following technical solutions.

A personnel on-duty status early-warning apparatus, including: an acquisition module, configured to continuously collect human body data of personnel in an actual working environment; a determination module, configured to determine on-duty status of the personnel corresponding to the collected human body data based on the obtained human body data by using a status detection model, wherein the status detection model is obtained by pre-training a preset model based on a training dataset, and the training dataset comprises human body data and corresponding on-duty status annotation information; a monitoring module, configured to perform on-duty status monitoring on the personnel based on the on-duty status determined for the personnel; and an output module, configured to output early-warning prompt information in response to the on-duty status of the personnel during the on-duty status monitoring satisfying a preset early-warning condition.

According to a third aspect, embodiments of the present disclosure provide an electronic device, adopting the following technical solutions.

The electronic device includes: at least one processor ; memory; and at least one application program. The at least one application program is stored in the memory and is configured to be executed by the at least one processor, and the at least one application program is configured to execute the personnel on-duty status early-warning method as described in the first aspect.

According to a fourth aspect, embodiments of the present disclosure provide a personnel on-duty status early-warning system, adopting the following technical solutions.

A personnel on-duty status early-warning system includes the electronic device as described in the third aspect and a wearable terminal, the wearable terminal is configured to collect the human body data and/or display the early-warning prompt information.

In one or more embodiments, the personnel on-duty status early-warning system further includes a simulation assembly, and the simulation assembly includes a simulation device and a collection device. The simulation device is configured for tested personnel to execute work tasks through the simulation device. The collection device is configured for collecting human body data of the tested personnel in a simulated working environment. The electronic device is further configured to obtain the input on-duty status annotation information and on-duty information.

According to a fifth aspect, embodiments of the present disclosure provide a computer-readable storage medium, adopting the following technical solutions.

A computer program is stored on the computer-readable storage medium, when the computer program is executed in a computer, the computer is instructed to execute the personnel on-duty status early-warning method as described in the first aspect.

To sum up, the present disclosure can achieve the following beneficial technical effects:

In the present disclosure, based on the obtained human body data of the personnel in the actual working environment, the on-duty status of the personnel can be determined by using the detection model. The state detection model is obtained by pre-training the preset model based on the training dataset, which can improve the detection accuracy of the on-duty status of the personnel. The training dataset includes human body data and its corresponding on-duty status annotation information, and the accurate subjective-perception information of the personnel can be obtained through the on-duty status annotation information, which improves the accuracy of performing the on-duty status monitoring on the personnel based on the on-duty status determined for the personnel. If the on-duty status of the personnel during the on-duty status monitoring meets the preset early-warning condition, the early-warning prompt information is output, thus avoiding the operation errors of the personnel and improving the life safety of the personnel.

### BRIEF DESCRIPTION OF DRAWINGS

Fig. 1 is a schematic flow diagram of a personnel on-duty status early-warning method according to an embodiment of the present disclosure;
Fig. 2 is a schematic structural diagram of a bracelet according to an embodiment of the present disclosure;
Fig. 3 is a simulation flow chart of a simulated working environment according to an embodiment of the present disclosure;
Fig. 4 is a schematic structural diagram of a personnel on-duty status early-warning system according to an embodiment of the present disclosure;
Fig. 5 is a schematic structural diagram of a personnel on-duty status early-warning apparatus according to an embodiment of the present disclosure; and
Fig. 6 is a schematic structural diagram of an electronic device according to an embodiment of the present disclosure.

### DESCRIPTION OF EMBODIMENTS

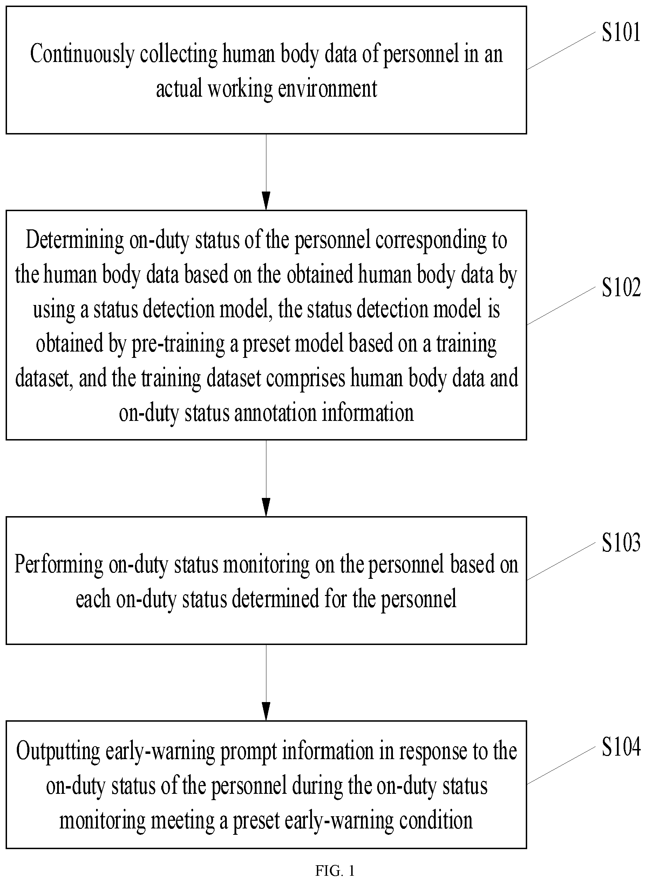

The present disclosure is explained in detail in conjunction with Figs. 1-6 as below.

Embodiments below are merely explanations of the present disclosure, which do not constitute any limitation to the present disclosure. Person skilled in the art can make modifications to the embodiments without creative efforts after reading the present disclosure, which all fall within the scope of the appended claims of the present disclosure and will be protected by the patent law.

To make the objectives, technical solutions and advantages of the embodiments of the present disclosure clearer, the technical solutions in the embodiments of the present disclosure will be described clearly and completely in conjunction with the accompanying drawings in the embodiments of the present disclosure. It is appreciated that the described embodiments are some of the embodiments of the present disclosure, rather than all of the embodiments. Based on the embodiments of the present disclosure, all other embodiments obtained by those of ordinary skill in the art without creative efforts fall within the protection scope of the present disclosure.

In addition, the term "and/or" in the context is merely a description of an associative relationship of the associated objects, indicating that there may be three relationships. For example, A and/or B may indicate: the presence of A alone, the presence of A and B at the same time, and the presence of B alone. In addition, the character "/" in the context, unless otherwise specified, generally indicates an "or" relationship between the associated objects.

An embodiment of the present disclosure provides a personnel on-duty status early-warning method. As shown in Fig. 1, the method is executed by an electronic device. The electronic device may be a server or a terminal device. The server may be an independent physical server, or a server cluster or distributed system composed of multiple physical servers, or a cloud server providing cloud computing services. The terminal device includes but is not limited to a smart phone, a tablet computer, a laptop computer, a desktop computer, etc. The terminal device and the server may be directly or indirectly connected through wired or wireless communication methods. Embodiments of the present disclosure do not have any limitation on the above forms or manners. The method includes steps S101-S104 as follows.

S101. Continuously collecting human body data of personnel in an actual working environment.

In some embodiments, a wearable terminal that the personnel needs to wear may be determined according to the personnel's working post, so as to collect the human body data of the personnel in the actual working environment through the wearable terminal. The human body data includes a variety of physiological data and/or electroencephalogram data. For the personnel in working posts with large movement range, such as construction workers, the wearable terminal they need to wear may be chest straps. For the personnel in working posts with small movement range, such as workshop operators, the wearable terminal they need to wear may be bracelets. For the personnel in working posts with small movement range and requiring mental labor, such as staff in a chemical laboratory, the wearable terminal they need to wear may be bracelets and electroencephalogram instruments.

In addition, different wearable terminals collect different human body data. The human body data that the bracelet and the chest strap can collect include: photoplethysmogram pulse wave signals (PPG), electrodermal activity (EDA), respiration signals (RESP), percutaneous arterial oxygen saturation (SpO2), skin temperature (SKT). In addition, the chest strap may further collect electrocardiogram signals (ECG), and the electroencephalogram instrument may collect electroencephalogram signals (EEG).

S 102. Determining on-duty status of the personnel corresponding to the human body data based on the obtained human body data by using the state detection model. The state detection model is obtained by pre-training a preset model based on a training dataset, and the training dataset includes human body data and its corresponding on-duty status annotation information.

In some embodiments, the preset model is pre-trained based on the human body data and subjective states of a large number of ordinary personnel and high-risk on-duty personnel. The pre-training process includes: setting multiple training scenarios, the training scenarios include cognitive load scenarios, fatigue scenarios and emotional scenarios, each training scenario includes training tasks corresponding to different levels, and the correspondence relationship between the levels and the training tasks is set artificially based on actual experience; collecting a large amount of human body data of multiple personnel performing the training tasks corresponding to different levels in each training scenario; performing model training by taking the human body data as the model input and taking the training scenarios and levels corresponding to the human body data as the model output. Optionally, the models include deep learning models, such as recurrent neural networks or long or short term memory networks.

The cognitive load scenarios, fatigue scenarios and emotional scenarios may be flexibly set according to actual requirements. Optionally, the cognitive load scenarios may be arithmetic, and the load scenarios of different levels correspond to arithmetic questions of different difficulties. The fatigue scenarios may be physical labor of different degrees. The emotional scenarios may be the emotional changes of the personnel in different scenarios.

Furthermore, the training dataset includes the human body data collected in the simulated working environment and its corresponding on-duty status annotation information and on-duty information, and/or the historical human body data collected in the actual working environment and its corresponding on-duty status annotation information and on-duty information. The on-duty information includes years of work and types of work. The types of work include high-risk types of work such as numerical control, construction, chemical engineering, and electrician. The tested personnel are multiple personnel corresponding to each type of work. Multiple training data may be obtained by acquiring the human body data and on-duty status annotation information of multiple tested personnel performing work tasks in the simulated working environment or the actual working environment. The preset model is trained based on multiple training data, and the state detection model that conforms to the physiological state of high-risk personnel may be obtained. The preset model is trained with a large amount of data and has powerful feature extraction and pattern recognition capabilities, which can greatly improve the performance of the tasks. Performing training and optimization based on the preset model can save a lot of training time and computing resources. The obtained state detection model can be directly applied to the on-duty status detection of high-risk personnel.

S103. Determining on-duty status monitoring of the personnel based on the on-duty status determined for the personnel.

In some embodiments, the on-duty status of the personnel obtained by using the human body data and the state detection model includes a cognitive load level, a fatigue level and an emotion level of the personnel.

S104. Outputting early-warning prompt information if the on-duty status of the personnel during the on-duty status monitoring meets a preset early-warning condition.

In some embodiments, according to the obtained on-duty status of the personnel, it is determined whether the on-duty status meets the preset early-warning condition. If yes, it indicates that the on-duty status is abnormal. If not, it indicates that the on-duty status is normal. The early-warning prompt information may be a rest instruction. In one possible situation, the rest instruction includes a rest duration to prompt the personnel to rest based on the rest duration indicated by the rest instruction. In another possible situation, the rest instruction does not include the rest duration, the rest instruction prompts the personnel to rest, and during the rest of the personnel, the human body data of the personnel is obtained in real time, and the state of the personnel during the rest is determined by using the state detection model. When the state of the personnel is a normal state, a working instruction may be sent to the terminal device of the personnel again to prompt the personnel to continue working.

After sending the early-warning prompt information to the terminal device of the high-risk personnel, a prompt signal corresponding to the abnormal state may also be generated. The prompt signal is sent to the terminal device corresponding to the management personnel, so as to prompt the management personnel to adjust the work tasks of the high-risk personnel. Optionally, the high-risk personnel may be adjusted to execute easier tasks next.

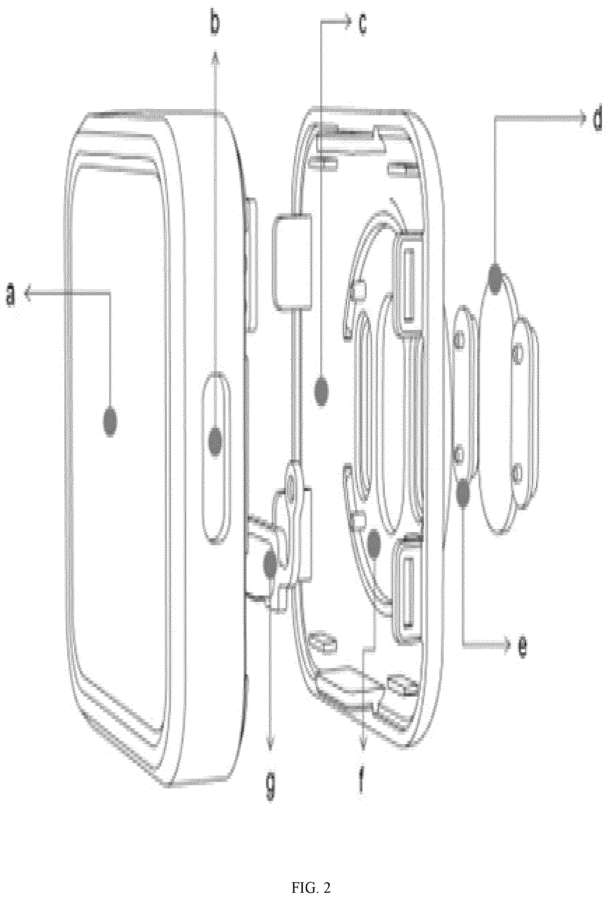

Fig. 2 shows a structural schematic diagram of a bracelet according to an embodiment of the present disclosure. The components of the bracelet include: display screen a, configured for real-time display of the physiological information of high-risk personnel; device charging port and device battery b, configured for at least eight hours of data collection and transmission; device lower shell c; vibration module d, configured for early-warning and reminder of abnormal states; Ag electrode e, configured for EDA signal collection; light source f, including three light sources of different wavelengths, green light may be configured for PPG signal collection, and red light and near-infrared light may be configured for SpO2 signal collection; device switch g; device upper shell h. The physiological data collected by the wearable terminal is transmitted to the electronic device in real time. The electronic device determines whether the on-duty status of the high-risk personnel is normal or abnormal based on the physiological data and transmits the on-duty status to the wearable terminal. If the on-duty status is abnormal, the bracelet vibrates to remind the high-risk personnel to rest.

In embodiments of the present disclosure, the on-duty status of the personnel may be determined by using the detection model based on the obtained human body data of the personnel in the actual working environment. The state detection model is obtained by pre-training the preset model based on the training dataset, which can improve the detection accuracy of the on-duty status of the personnel. The training dataset includes human body data and its corresponding on-duty status annotation information, and the accurate subjective-perception information of the personnel may be obtained through the on-duty status annotation information, which improves the accuracy of performing on-duty status monitoring on the personnel based on the on-duty status determined for the personnel. If the on-duty status of the personnel during the on-duty status monitoring meets the preset early-warning condition, the early-warning prompt information is output, avoiding the operation errors of the personnel and thus improving the life safety of the personnel.

In some embodiments, the method further includes: determining work tasks, interactive elements, control methods and feedback mechanisms; building a simulated working environment based on the work tasks, interactive elements, control methods and feedback mechanisms; generating task instructions and sending the task instructions to a terminal device of tested personnel to prompt the tested personnel to execute the work tasks corresponding to the task instructions in the simulated working environment; obtaining human body data corresponding to the tested personnel when executing the work tasks and its corresponding on-duty status annotation information and on-duty information; generating the training dataset based on the human body data corresponding to the tested personnel and its corresponding on-duty status annotation information and on-duty information; and training the preset model through the training dataset to obtain the state detection model.

In some embodiments, the types of work may be determined based on the working environments of high-risk jobs. For indoor high-risk jobs such as numerical control and chemical engineering, it is determined that the corresponding type of work may be a manufacturing type; for outdoor high-risk jobs such as construction and electrician, it is determined that the corresponding type of work may be a construction type.

Further, a simulated working environment corresponding to the type of work may be built. For the manufacturing type of working environment, a real operation console may be built to simulate the operations in high-risk jobs of the manufacturing type. For the construction type, a virtual reality environment (VRE) may be built, and work tasks of real construction type may simulated through VR equipment (such as VR glasses) using the virtual reality environment.

A database may be pre-established and stored in the electronic device. The database includes the correspondence between the identities of the tested personnel and their working posts, as well as the correspondence between the working posts and the work tasks. After determining the identity of the tested personnel, the corresponding working task of the tested personnel may be retrieved from the database. Task instructions are generated based on the working task and sent to the terminal device of the tested personnel to prompt the tested personnel to execute the corresponding working task based on the task instructions.

Specifically, for the manufacturing type, the interactive elements included in the operation console may be set based on the working contents of the working posts corresponding to the manufacturing type. The interactive elements may be flexibly set according to the working contents. Optionally, the interactive elements may include keys, knobs and handles, etc. According to the working contents in the actual high-risk working environment, the interactive process corresponding to the interactive elements is set as the working contents. For example, for numerical control workers, the operation steps of keys, knobs and handles may be set as the working contents corresponding to numerical control workers. The control methods of the simulated working environment corresponding to the manufacturing type are the control through the interactive elements of the operation console. Furthermore, a screen or projector may be set to display the process of the tested personnel performing the work tasks and to feedback the execution result of the work tasks.

For the construction type of working environment, the work tasks may include operations such as grasping, moving and interacting. The interactive elements may include virtual objects in the virtual reality environment, which are used by the tested personnel to interact with the virtual objects through the virtual reality environment. The control methods of the construction type are the interaction through the virtual reality environment. A background display screen may be set to display the result pictures of executing the work tasks in the virtual reality environment.

During the process of the tested personnel performing the work tasks in the simulated working environment, a collection device corresponding to the working post is worn to collect the physiological data and on-duty status annotation information of the tested personnel when executing the work tasks through the collection device. The on-duty status annotation information of the tested personnel includes fatigue data and emotion data, which are used to indicate the fatigue degree and emotion of the tested personnel. The collection device may be the same as the wearable terminal or different from the wearable terminal.

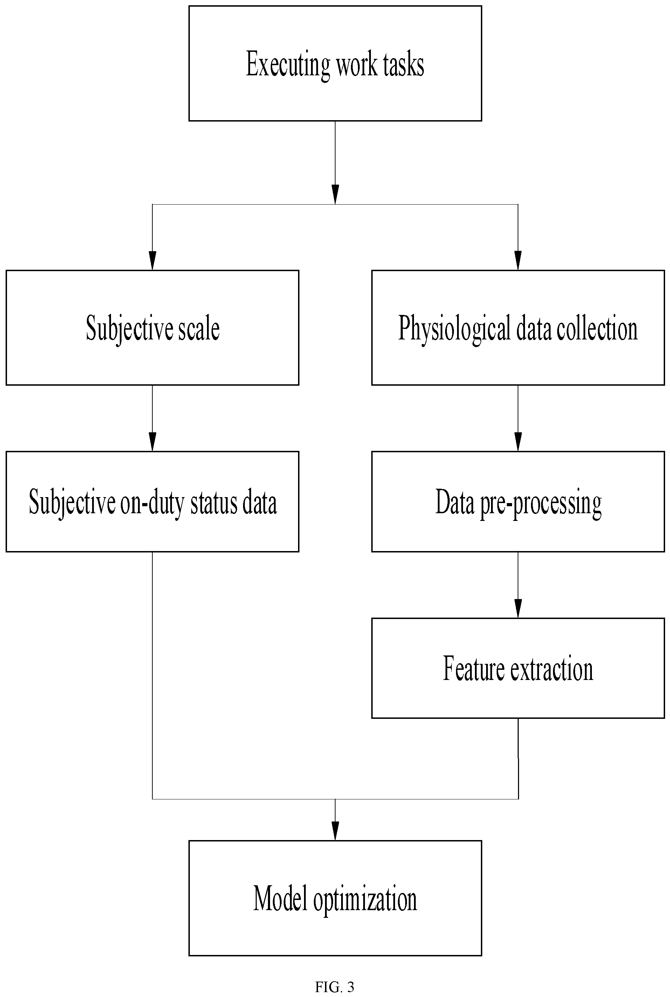

Fig. 3 shows a simulation flow chart of a simulated working environment according to an embodiment of the present disclosure. The tested personnel executes the corresponding work tasks in the simulated working environment. The collection device worn by the tested personnel collects the physiological data during the process of the tested personnel executing the work tasks. Data pre-processing and feature extraction are performed on the physiological data. Data pre-processing may include filtering and noise reduction, and the extracted features may include time domain features, frequency domain features and nonlinear features. Based on the preset subjective scale, and the scale conclusion data corresponding to the tested personnel is obtained as the on-duty status annotation information of the tested personnel. The extracted features and on-duty status annotation information are input into the preset model for model optimization, and an optimized state detection model suitable for high-risk personnel may be obtained.

Due to long-term concentration and high-intensity work load of the high-risk personnel, the load level, psychological ability and fatigue state of high-risk personnel are different from those of ordinary personnel. The preset model is trained based on the data of ordinary personnel. When ordinary personnel already feels high load, high-risk personnel may still be in a normal state. Therefore, in order to make the algorithm model more accurate for the on-duty status detection of the high-risk personnel, subjective scale experiments may be conducted during the rest stage after the tested personnel executes the work tasks in the simulated working environment, the on-duty status annotation information of the tested personnel is obtained and recorded. The on-duty status annotation information is input into the preset model as the label of the physiological data to make the model adaptively optimize, thereby continuously improving the professionalism of the model, and making it a state detection model suitable for the high-risk personnel.

In embodiments of the present disclosure, by building a simulated working environment, the tested personnel performs the work tasks through the simulated working environment, so that the human body data of the tested personnel in the simulated working environment and its corresponding on-duty status annotation information and on-duty information are obtained, improving the accuracy of determining the training dataset. By training the preset model through the training dataset, a state detection model with more accurate detection is obtained, which improves the test accuracy of the state detection model.

In some embodiments, the training dataset includes a training set, a test set and a validation set.

The training the preset model through the training dataset to obtain the state detection model, includes: performing initial training on the preset model based on the training set to obtain an initial model, the preset model is obtained by being pre-trained based on an initial training dataset, and the initial training dataset at least includes human body data of ordinary personnel and its corresponding on-duty status annotation information; performing metric testing on the initial model based on the test set to obtain an initial state detection model; and verifying the initial state detection model based on the validation set to obtain the state detection model.

In some embodiments of the present disclosure, through the training set, test set and validation set, the preset model is initially trained, tested and verified in sequence. The obtained state detection model is more in line with the physical state of the personnel in the working environment, improving the detection accuracy of the state detection model.

In some embodiments, the obtaining the human body data corresponding to the tested personnel when executing the work tasks and its corresponding on-duty status annotation information and on-duty information, includes: during the process of the tested personnel executing the work tasks, obtaining the human body data corresponding to the tested personnel, a working duration of the tested personnel, and limb movement data of the tested personnel; inputting the human body data corresponding to the tested personnel into the preset model to generate a simulated on-duty status of the tested personnel; determining whether the simulated on-duty status is an abnormal state, whether the working duration reaches a preset duration, and/or whether limb movements of the tested personnel are qualified based on the limb movement data; and generating on-duty status annotation information and on-duty information of the tested personnel based on the determined result.

In embodiments of the present disclosure, during the process of the tested personnel executing the work tasks, the collection device worn by the tested personnel collects the human body data of the tested personnel in real time, and inputs the real-time collected human body data into the preset model. The preset model may determine the on-duty status of the tested personnel executing the work tasks in the simulated working environment based on the real-time human body data. The on-duty status includes a cognitive load level, a fatigue level and an emotion level of the tested personnel. According to the cognitive load level, fatigue level and emotion level of the tested personnel, it may be determined whether the simulated on-duty status is abnormal. The preset duration may be set according to actual requirements. Optionally, the preset duration may be 2 hours.

The database pre-stores the qualified operations corresponding to each working task. During the process of the tested personnel executing the work tasks in the simulated working environment, the limb movements of the tested personnel are compared with the corresponding qualified operations in the database. If the contents corresponding to the work tasks are to control the operation console, the comparison contents may be whether the order of steps for controlling the operation console conforms to the corresponding qualified operations. If the work contents are grasping, moving or operating with other objects in the virtual reality environment, the comparison contents may be whether the grasping is successful, whether it is moved to the designated position, and whether the interaction is successful, thereby determining whether the limb movements of the tested personnel are qualified.

Further, when the simulated on-duty status is abnormal, or the working duration reaches the preset duration, or the operations of the tested personnel are unqualified, a stop execution signal is generated. The stop execution signal may include a stop instruction and a rest instruction. The stop instruction is used to instruct the tested personnel to stop the work tasks, and the rest instruction is used to prompt the tested personnel to rest for a certain duration. The certain duration is preset according to actual requirements. Optionally, the certain duration may be 10 minutes. During the rest period of the tested personnel, the on-duty status annotation information of the tested personnel may be collected. In addition, the physiological data of the tested personnel is collected in real time and it is determined whether there is an abnormal state through the preset model. When the state of the tested personnel is normal and the rest duration reaches a certain duration, a continue working instruction may be sent to the tested personnel to prompt the tested personnel to return to the simulated working environment, so as to continue executing the work tasks. In order to simulate the real working environment, the total simulation duration may be set to 8 hours, including the working duration of executing work tasks in the simulated working environment and the rest duration in the middle.

In embodiments of the present disclosure, by determining whether the simulated on-duty status is an abnormal state, whether the working duration reaches the preset duration, and/or whether the limb movements of the tested personnel are qualified based on the limb movement data, and generating the on-duty status annotation information and on-duty information of the tested personnel based on the determined result, the accuracy of the on-duty status annotation information is improved.

In some embodiments, the on-duty status annotation information at least includes fatigue data and/or emotion data.

The generating on-duty status annotation information of the tested personnel, includes: sending a fatigue assessment scale and/or an emotion scale to the terminal device of the tested personnel through a subjective questionnaire; and receiving fatigue data of the fatigue assessment scale and/or emotion data of the emotion scale corresponding to the tested personnel.

In some embodiments, the fatigue assessment scale may adopt the rating of perceived exertion (RPE) to collect the fatigue degree of the tested personnel. The process includes that the tested personnel selects the score that is most in line with the current state as the fatigue data of the fatigue assessment scale corresponding to the tested personnel. Optionally, the score range is 6-20. The larger the value is, the more fatigue the subjective perception of the tested personnel is. The value 6 indicates extremely low fatigue feeling, and the value 20 indicates extremely high fatigue feeling.

The emotion scale may adopt the Pleasure-Arousal-Dominance (PAD) emotion scale to measure and evaluate the emotions of the tested personnel. The process is to set multiple questions. Each question contains a group of adjectives. One group consists of two adjectives, regardless of good or bad. The two adjectives are placed at both ends, and the middle of the two adjectives is divide into nine grades. They may be evaluated with scores from -4 to 4. The tested personnel may select the corresponding score according to the current state to indicate the current feeling. Alternative adjective groups include: angry and lively, sleepy and awake, externally controlled and self-controlled, contemptuous and friendly, calm and excited, compliant and dominant, painful and happy, relaxed and interested, guided and dominant, irritated and excited, relaxed and hopeful, lively and influential.

The simplified Chinese version of the PAD emotion scale may be adopted, which contains 12 questions and adopts a nine-level scoring system. Questions 2, 4, 6, 8, 10 and 12 are reverse scoring questions. Based on the score of each question selected by the tested personnel, the emotion data of the emotion scale corresponding to the tested personnel may be obtained. The emotion data includes three dimensions: pleasure degree, activation degree and dominance degree. The pleasure degree may reflect the positive and negative characteristics of the emotional state of the tested personnel, the activation degree may reflect the neurophysiological activation level of the tested personnel, and the dominance degree may reflect the control state of the tested personnel over the situation and others. Through the three dimensions, it may be evaluated whether the current state of the tested personnel meets the work requirements.

In some embodiments of the present disclosure, through the fatigue assessment scale and the emotion scale, the accurate fatigue data and emotion data of the tested personnel may be obtained, improving the accuracy of the on-duty status annotation information. The subjective on-duty status of the tested personnel in multiple aspects may be evaluated through the fatigue data and emotion data.

In some embodiments, the determining on-duty status of the personnel corresponding to the human body data based on the obtained human body data by using the state detection model, includes: performing data pre-processing and feature extraction on the human body data; inputting the extracted features into the state detection model to determine fatigue data and emotion data corresponding to the human body data, the on-duty status annotation information in the training dataset corresponding to the state detection model includes fatigue data and emotion data; and inputting the fatigue data and emotion data into a preset classification model to obtain on-duty status of the personnel corresponding to the human body data, the on-duty status includes a cognitive load level, a fatigue level and an emotion level.

In some embodiments, data pre-processing may include filtering and noise reduction. The methods of filtering and noise reduction may be median filtering method, and low-pass digital filtering method, etc., which is not specifically limited by some embodiments. The features extracted from the physiological data to be detected may include: time series data (such as the data of ECG, EEG, and RESP changing over time), frequency features, waveform features, time domain features (such as the mean, variance and peak value of the signal), frequency domain features (such as spectrum and power spectrum).

Specifically, the optional levels corresponding to cognitive load, fatigue and emotion may be set according to actual requirements. Alternatively, the cognitive load levels may include high cognitive load level, medium cognitive load level and low cognitive load level; the fatigue levels may include high fatigue level, medium fatigue level and low fatigue level; the emotion levels may include high emotion level, medium emotion level and low emotion level. The higher the level is, the higher the corresponding degree is. The preset classification model is used to generate the corresponding on-duty status based on the fatigue data and emotion data. The preset classification model may be a machine learning model. The input in the training process of the preset classification model is the fatigue data and emotion data, and the output is the on-duty status of the personnel.

In embodiments of the present disclosure, by performing data pre-processing, feature extraction and state detection on the human body data, the fatigue data and emotion data corresponding to the human body data may be determined. The fatigue data and emotion data are input into the preset classification model, improving the accuracy of determining the on-duty status of the personnel.

In some embodiments, the determining on-duty status of the personnel corresponding to the human body data based on the obtained human body data by using a state detection model, includes: performing data pre-processing and feature extraction on the human body data; and inputting the extracted features into the state detection model to determine the on-duty status of the personnel corresponding to the human body data, the on-duty status annotation information in the training dataset corresponding to the state detection model includes cognitive load level annotation information, fatigue level annotation information and emotion level annotation information.

In embodiments of the present disclosure, by inputting the extracted features into the state detection model, the on-duty status of the personnel corresponding to the human body data is determined, and the on-duty status annotation information in the training dataset corresponding to the state detection model includes cognitive load level annotation information, fatigue level annotation information and emotion level annotation information.

In some embodiments, the performing on-duty status monitoring on the personnel based on the on-duty status determined for the personnel , includes: determining whether the cognitive load level, fatigue level and emotion level exceed their respective corresponding level thresholds; and determining that the on-duty status of the personnel does not meet the preset early-warning condition if all of the cognitive load level, fatigue level and emotion level do not exceed the corresponding level thresholds, or determining that the on-duty status of the personnel meets the preset early-warning condition if any one of the cognitive load level, fatigue level and emotion level exceeds its corresponding level threshold.

In some embodiments, the medium cognitive load level may be used as the level threshold corresponding to the cognitive load level, the medium fatigue level may be used as the level threshold corresponding to the fatigue level, and the medium emotion level may be used as the level threshold corresponding to the emotion level.

In embodiments of the present disclosure, according to the comparison results of the cognitive load level, fatigue level and emotion level with the respective corresponding level thresholds, it may be determined whether the on-duty status of the high-risk personnel meets the preset early-warning condition, improving the determination efficiency and accuracy.

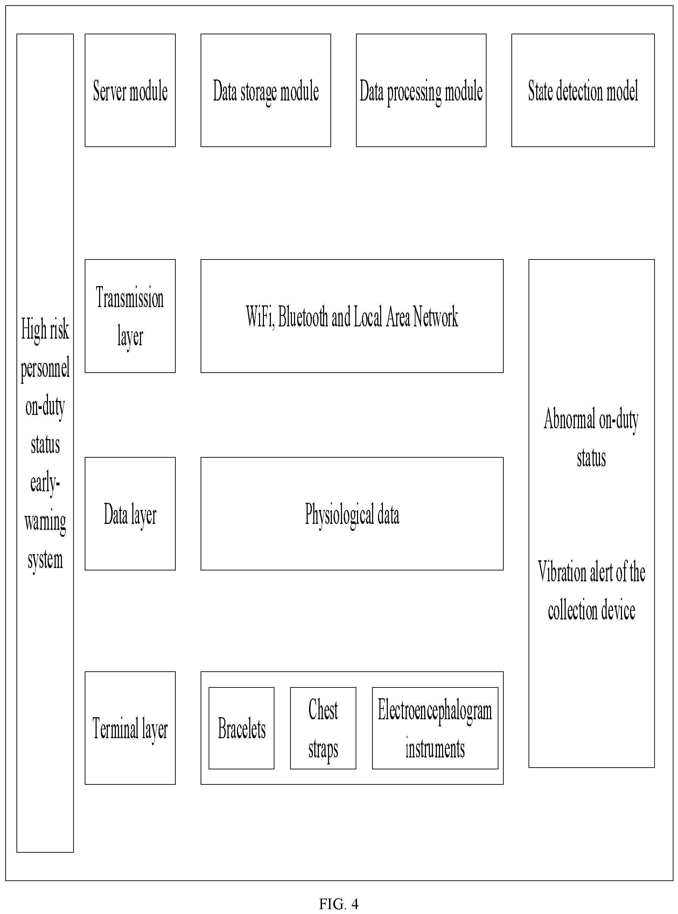

Fig. 4 shows a structural schematic diagram of a personnel on-duty status early-warning system according to an embodiment of the present disclosure. The system includes the terminal layer and an electronic device. The electronic device includes the data layer, the transmission layer and the server module. The terminal layer may be wearable terminals, and the wearable terminals include bracelets, chest straps and electroencephalogram instruments. The data layer includes the human body data collected by the wearable terminals. The collected human body data may be transmitted to the server module through the transmission layer. Optional transmission methods include Wireless Fidelity (Wi-Fi), Bluetooth and local area network. The server module receives the human body data transmitted by the transmission layer. The server module includes a data storage module, a data processing module and a state detection model. The data storage module contains a database, and the database includes user management, which stores the correspondence between the identifiers (IDs) of the wearable terminals and the identity information of the personnel wearing the wearable terminal. The identity information of the personnel corresponding to the human body data may be determined based the ID of the wearable terminal corresponding to the received human body data. The data processing module may perform pre-processing and feature extraction on the received human body data. The state detection model may determine whether the on-duty status of the personnel is normal or abnormal based on the extracted features. If the on-duty status of the personnel is abnormal, the personnel may be reminded to rest through the vibration of the wearable terminal.

The above embodiments introduce personnel on-duty status early-warning methods from the perspective of the method process. The following embodiments introduce a personnel on-duty status early-warning apparatus from the perspective of virtual modules or virtual units. For details, please refer to the following embodiments.

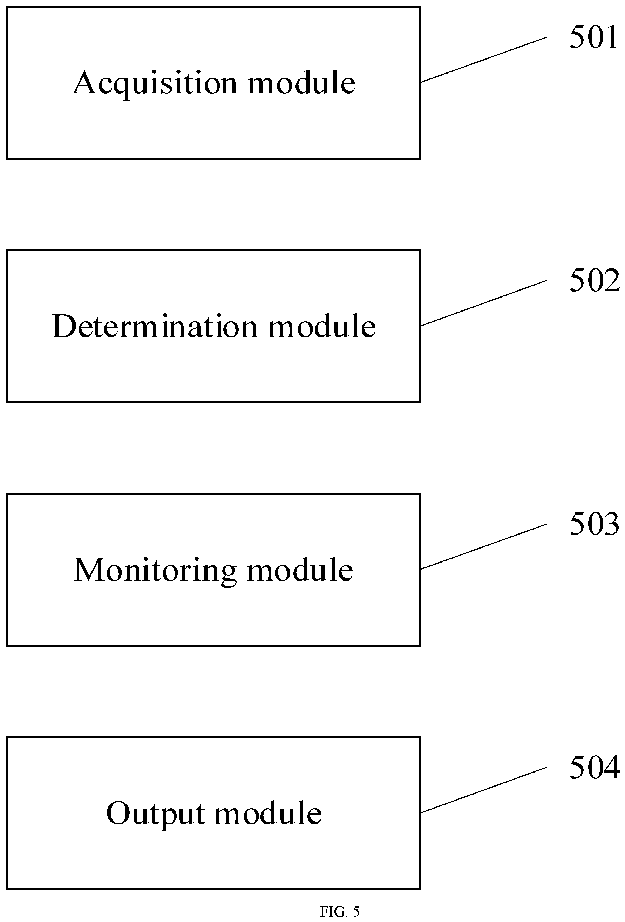

An embodiment of the present disclosure provides a personnel on-duty status early-warning apparatus. As shown in Fig. 5, the apparatus may include: an acquisition module 501, configured to acquire human body data of personnel in an actual working environment continuously; a determination module 502, configured to determine on-duty status of the personnel corresponding to the human body data based on the obtained human body data by using a state detection model, the state detection model is obtained by pre-training a preset model based on a training dataset, and the training dataset includes human body data and its corresponding on-duty status annotation information; a monitoring module 503, configured to perform on-duty status monitoring on the personnel based on the on-duty status determined for the personnel; and an output module 504, configured to output early-warning prompt information if the on-duty status of the personnel during the on-duty status monitoring meets a preset early-warning condition.

In some embodiments, the apparatus further includes a simulation module, which is specifically configured to: determine work tasks, interactive elements, control methods and feedback mechanisms; build a simulated working environment based on the work tasks, interactive elements, control methods and feedback mechanisms; generate task instructions and send the task instructions to a terminal device of tested personnel to prompt the tested personnel to execute the work tasks corresponding to the task instructions in the simulated working environment; obtain human body data corresponding to the tested personnel when executing the work tasks and its corresponding on-duty status annotation information and on-duty information; generate the training dataset based on the human body data and its corresponding on-duty status annotation information and on-duty information; and train the preset model through the training dataset to obtain the state detection model.

In some embodiments, when the simulation module performs training on the preset model through the training dataset to obtain the state detection model, it is specifically configured to: perform initial training on the preset model based on the training set to obtain an initial model, the preset model is obtained by being pre-trained based on an initial training dataset, and the initial training dataset at least includes human body data of ordinary personnel and its corresponding on-duty status annotation information; perform metric testing on the initial model based on the test set to obtain an initial state detection model; and verify the initial state detection model based on the validation set to obtain the state detection model.

In some embodiments, when the simulation module performs obtaining human body data corresponding to the tested personnel when executing the work tasks and its corresponding on-duty status annotation information and on-duty information, it is specifically configured to: during the process of the tested personnel performing the work tasks, obtain the human body data corresponding to the tested personnel, a working duration of the tested personnel, and limb movement data of the tested personnel; input the human body data into the preset model to generate a simulated on-duty status of the tested personnel; determine whether the simulated on-duty status is an abnormal state, whether the working duration reaches a preset duration, and/or whether limb movements of the tested personnel are qualified based on the limb movement data; and generate on-duty status annotation information and on-duty information of the tested personnel based on the determined result.

In some embodiments, when the simulation module performs generating on-duty status annotation information of the tested personnel, it is specifically configured to: send a fatigue assessment scale and/or an emotion scale to the terminal device of the tested personnel through a subjective questionnaire; and receive fatigue data of the fatigue assessment scale and/or emotion data of the emotion scale corresponding to the tested personnel.

In some embodiments, when the determination module 502 performs determining on-duty status of the personnel corresponding to the human body data based on the obtained human body data by using a state detection model, it is specifically configured to: perform data pre-processing and feature extraction on the human body data; input the extracted features into the state detection model to determine fatigue data and emotion data corresponding to the human body data, the on-duty status annotation information in the training dataset corresponding to the state detection model includes fatigue data and emotion data; and input the fatigue data and emotion data into a preset classification model to obtain the on-duty status of the personnel corresponding to the human body data, the on-duty status includes a cognitive load level, a fatigue level and an emotion level.

In one or more embodiments, when the determination module 502 performs determining on-duty status of the personnel corresponding to the human body data based on the obtained human body data by using the state detection model, it is specifically configured to: perform data pre-processing and feature extraction on the human body data; and input the extracted features into the state detection model to determine the on-duty status of the personnel corresponding to the human body data, the on-duty status annotation information in the training dataset corresponding to the state detection model includes cognitive load level annotation information, fatigue level annotation information and emotion level annotation information.

In some embodiments, when the monitoring module 503 performs on-duty status monitoring of the personnel based on the on-duty status determined for the personnel, it is specifically configured to: determine whether the cognitive load level, fatigue level and emotion level exceed their respective corresponding level thresholds; and determine that the on-duty status of the personnel does not meet the preset early-warning condition if all of the cognitive load level, fatigue level and emotion level do not exceed their respective corresponding level thresholds, or determine that the on-duty status of the personnel meets the preset early-warning condition if any one of the cognitive load level, fatigue level and emotion level exceeds its corresponding level threshold.

The personnel on-duty status early-warning apparatus according to the embodiments of the present disclosure is applicable to the above method embodiments and will not be repeated here.

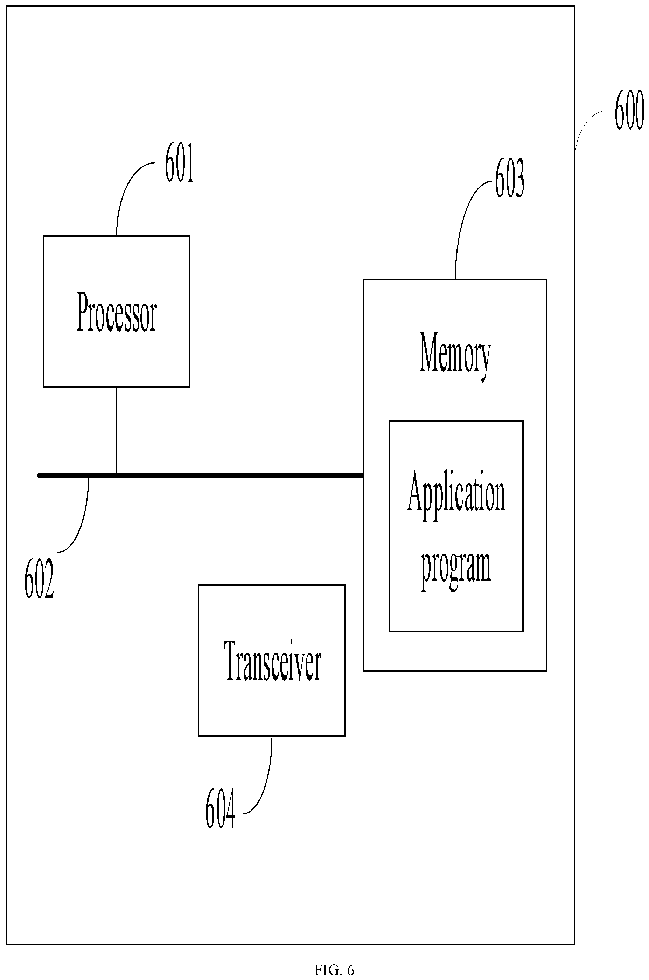

An embodiment of the present disclosure provides an electronic device. As shown in Fig. 6, the electronic device 600 shown in Fig. 6 includes: a processor 601 and a memory 603. The processor 601 and the memory 603 are connected, such as connected through a bus 602. Optionally, the electronic device 600 may also include a transceiver 604. It should be noted that the transceiver 604 is not limited to one in practical applications, and the structure of the electronic device 600 does not constitute any limitation to the embodiments of the present disclosure.

The processor 601 may be a Central Processing Unit (CPU), a general-purpose processor, a Digital Signal Processor (DSP), an Application Specific Integrated Circuit (ASIC), an Field Programmable Gate Array (FPGA), or other programmable logic devices, transistor logic devices, hardware components, or any combination thereof. It may implement or execute various exemplary logic blocks, modules and circuits described in combination with the content disclosed in the present disclosure. The processor 601 may also be a combination for realizing computing functions, such as a combination of one or more microprocessors, a combination of a DSP and a microprocessor, etc.

The bus 602 may include a path for transmitting information between the above components. The bus 602 may be a Peripheral Component Interconnect (PCI) bus or an Extended Industry Standard Architecture (EISA) bus, etc. The bus 602 may be divided into an address bus, a data bus, a control bus, etc. For ease of representation, only one thick line is shown in Fig. 6, but it does not mean that there is only one bus or one type of bus.

The memory 603 may be a Read Only Memory (ROM) or other types of static storage devices that can store static information and instructions, a Random Access Memory (RAM) or other types of dynamic storage devices that can store information and instructions. It may also be an Electrically Erasable Programmable Read Only Memory (EEPROM), a Compact Disc Read Only Memory (CD-ROM) or other optical disc storage, optical disk storage (including compact discs, laser discs, optical discs, digital versatile discs, Blu-ray discs, etc.), magnetic disk storage media or other magnetic storage devices, or any other medium that can be used to carry or store desired program code in the form of instructions or data structures and can be accessed by a computer, but is not limited to this.

The memory 603 is used to store the application program code for implementing the present application solutions and is controlled by the processor 601 for execution. The processor 601 is used to execute the application program code stored in the memory 603 to implement the contents described in the foregoing embodiments of the personnel on-duty status early-warning method.

The electronic device shown in Fig. 6 is only an example and should not bring any limitation to the function and use scope of the embodiments of the present disclosure.

An embodiment of the present disclosure provides a personnel on-duty status early-warning system, including the above electronic device and the wearable terminal. The wearable terminal is configured to collect the human body data and/or display the early-warning prompt information. The personnel on-duty status early-warning system may further include a simulation assembly. The simulation assembly includes a simulation device and a collection device. The simulation device is configured for the tested personnel to execute work tasks through the simulation device. The collection device is configured for collecting the human body data of the tested personnel in the simulated working environment. The electronic device is further configured for obtaining the input on-duty status annotation information and on-duty information.

An embodiment of the present disclosure provides a computer-readable storage medium on which a computer program is stored, when the computer program is executed in a computer, the computer is instructed to execute the corresponding contents in the foregoing method embodiments.

It should be understood that although the various steps in the flowcharts of the accompanying drawings are displayed in sequence as indicated by the arrows, these steps are not necessarily executed in the order indicated by the arrows. Unless explicitly stated herein, there are no strict order restrictions for the execution of these steps, and they may be executed in other orders. Moreover, at least some of the steps in the flowcharts of the accompanying drawings may include multiple sub-steps or multiple stages. These sub-steps or stages are not necessarily executed at the same time, but may be executed at different times. Their execution order is not necessarily sequential, but may be executed alternately or in turn with at least some of the other steps or sub-steps or stages of the other steps.

The above are only some of the embodiments of the present disclosure. The scope of the present disclosure shall be defined by the appended claims.

## Claims

1. A personnel on-duty status early-warning method, comprising: continuously collecting human body data of personnel in an actual working environment; determining on-duty status of the personnel corresponding to the human body data based on the collected human body data by using a status detection model, wherein the status detection model is obtained by pre-training a preset model based on a training dataset, and the training dataset comprises human body data and corresponding on-duty status annotation information;performing on-duty status monitoring on the personnel based on the on-duty status determined for the personnel; and outputting early-warning prompt information in response to the on-duty status of the personnel during the on-duty status monitoring satisfying a preset early-warning condition.

2. The personnel on-duty status early-warning method according to claim 1, wherein the training dataset comprises human body data collected in a simulated working environment, and corresponding on-duty status annotation information and on-duty information, and/or, the training dataset comprises historical human body data collected in an actual working environment and corresponding on-duty status annotation information and on-duty information; and wherein the on-duty information comprises years of work and types of work.

3. The personnel on-duty status early-warning method according to claim 2, further comprising: determining work tasks, interactive elements, control methods and feedback mechanisms; building the simulated working environment based on the work tasks, the interactive elements, the control methods and the feedback mechanisms; generating task instructions and sending the task instructions to a terminal device of tested personnel to prompt the tested personnel to execute the work tasks corresponding to the task instructions in the simulated working environment; obtaining human body data corresponding to the tested personnel when executing the work tasks and corresponding on-duty status annotation information and on-duty information; generating the training dataset based on the human body data corresponding to the tested personnel and corresponding on-duty status annotation information and on-duty information; and training the preset model with the training dataset to obtain the status detection model.

4. The personnel on-duty status early-warning method according to claim 3, wherein the training dataset comprises a training set, a test set and a validation set, and the training the preset model with the training dataset to obtain the status detection model, comprises:performing initial training on the preset model to obtain an initial model based on the training set, wherein the preset model is obtained by pre-training based on an initial training dataset, and the initial training dataset at least comprises human body data of ordinary personnel and corresponding on-duty status annotation information; performing metric testing on the initial model based on the test set to obtain an initial status detection model; and verifying the initial status detection model based on the validation set to obtain the status detection model.

5. The personnel on-duty status early-warning method according to claim 3, wherein the obtaining human body data corresponding to the tested personnel when executing the work tasks and corresponding on-duty status annotation information and on-duty information, comprises:
during the process of the tested personnel executing the work tasks, obtaining the human body data corresponding to the tested personnel, a working duration of the tested personnel, and limb movement data of the tested personnel; inputting the human body data corresponding to the tested personnel into the preset model to generate simulated on-duty status of the tested personnel;
determining whether the simulated on-duty status is an abnormal state, whether the working duration reaches a preset duration, and/or whether limb movements of the tested personnel are qualified based on the limb movement data; and generating the on-duty status annotation information and on-duty information of the tested personnel based on the determination result.

6. The personnel on-duty status early-warning method according to claim 5, wherein the on-duty status annotation information at least comprises fatigue data and/or emotion data, and
the generating the on-duty status annotation information of the tested personnel, comprises:
sending a fatigue assessment scale and/or an emotion scale to the terminal device of the tested personnel through a subjective questionnaire; and receiving fatigue data of the fatigue assessment scale and/or emotion data of the emotion scale corresponding to the tested personnel.

7. The personnel on-duty status early-warning method according to claim 1, wherein the determining on-duty status of the personnel corresponding to the human body data based on the obtained human body data by using a status detection model, comprises: performing data pre-processing and feature extraction on the human body data; inputting the extracted features into the status detection model to determine fatigue data and emotion data corresponding to the human body data, wherein the on-duty status annotation information in the training dataset corresponding to the status detection model comprises fatigue data and emotion data; and inputting the fatigue data and emotion data into a preset classification model to obtain the on-duty status of the personnel corresponding to the human body data, wherein the on-duty status comprises a cognitive load level, a fatigue level and an emotion level.

8. The personnel on-duty status early-warning method according to claim 1, wherein the determining on-duty status of the personnel corresponding to the human body data based on the obtained human body data, comprises: performing data pre-processing and feature extraction on the human body data; and inputting the extracted features into the status detection model to determine the on-duty status of the personnel corresponding to the human body data, wherein the on-duty status annotation information in the training dataset corresponding to the status detection model comprises cognitive load level annotation information, fatigue level annotation information and emotion level annotation information.

9. The personnel on-duty status early-warning method according to claim 8, wherein the performing on-duty status monitoring on the personnel based on the on-duty status determined for the personnel, comprises: determining whether the cognitive load level, fatigue level and emotion level exceed respective level thresholds; and determining that the on-duty status of the personnel does not meet the preset early-warning condition in response to all of the cognitive load level, fatigue level and emotion level do not exceed respective level thresholds, or determining that the on-duty status of the personnel meets the preset early-warning condition in response to any one of the cognitive load level, fatigue level and emotion level exceeds respective level threshold.

10. The personnel on-duty status early-warning method according to claim 1, wherein the human body data comprises a variety of physiological data and/or electroencephalogram data.

11. A personnel on-duty status early-warning apparatus, comprising:
an acquisition module, configured to continuously collect human body data of personnel in an actual working environment; a determination module, configured to determine on-duty status of the personnel corresponding to the collected human body data based on the obtained human body data by using a status detection model, wherein the status detection model is obtained by pre-training a preset model based on a training dataset, and the training dataset comprises human body data and corresponding on-duty status annotation information; a monitoring module, configured to perform on-duty status monitoring on the personnel based on the on-duty status determined for the personnel; and an output module, configured to output early-warning prompt information in response to the on-duty status of the personnel during the on-duty status monitoring satisfying a preset early-warning condition.

12. An electronic device, comprising: at least one processor; a memory; and at least one application program stored in the memory and executed by the at least one processor, wherein the at least one application program is configured to execute the personnel on-duty status early-warning method according to any one of claims 1-10.

13. A personnel on-duty status early-warning system, comprising: the electronic device according to claim 12 and a wearable terminal, wherein the wearable terminal is configured to collect the human body data and/or display the early-warning prompt information.

14. The personnel on-duty status early-warning system according to claim 13, further comprising: a simulation assembly, and wherein the simulation assembly comprises: a simulation device configured for tested personnel to execute work tasks through the simulation device; and a collection device configured for collecting the human body data of the tested personnel in a simulated working environment; wherein the electronic device is further configured to obtain the input on-duty status annotation information and on-duty information.

15. A non-transitory computer-readable storage medium on which a computer program is stored, wherein when the computer program is executed in a computer, the computer is instructed to execute the personnel on-duty status early-warning method according to any one of claims 1-10.
